# FASCICULE DE BREVET EUROPEEN

(11) **EP 2 076 585 B1**
(45) Date de publication et mention de la délivrance du brevet: **03.09.2014**
(21) Numéro de dépôt: 07820928.5
(22) Date de dépôt: 04.10.2007
(51) Int. Cl.: C12N 1/20, C12P 7/66, A23C 9/123, A23L 1/302

(54) **PROCEDE DE CULTURE POUR FAVORISER LA PRODUCTION DE VITAMINE K2 PAR LES BACTERIES LACTIQUES ET SES APPLICATIONS A LA PREPARATION DE PRODUITS ALIMENTAIRES**
KULTURVERFAHREN ZUR BEGÜNSTIGUNG DER PRODUKTION VON VITAMIN K2 DURCH MILCHSÄUREBAKTERIEN SOWIE ANWENDUNGEN DAVON BEI DER HERSTELLUNG VON LEBENSMITTELPRODUKTEN
CULTURE METHOD FOR FAVORISING THE PRODUCTION OF VITAMIN K2 BY LACTIC ACID BACTERIA AND APPLICATIONS THEREOF IN THE PREPARATION OF FOOD PRODUCTS

(30) Priorité: 04.10.2006 FR 0608692
(43) Date de publication de la demande: 08.07.2009
(73) Titulaire: Compagnie Gervais Danone, 75009 Paris (FR)
(72) Inventeur: GARAULT, Peggy, 91310 Montlhery (FR); QUERE, Gaëlle, 91140 Villebon Sur Yvette (FR); CATONNET, Guillaume, 91300 Massy (FR); LAMICHE, Chantal, 91370 Verrieres Le Buisson (FR); FAURIE, Jean-Michel, 78350 Jouy en Josas (FR)
(74) Mandataire: Regimbeau
(86) Numéro de dépôt international: PCT/EP2007/060551
(87) Numéro de publication internationale: WO 2008/040784

(56) Documents cités:
- WO-A1-00/05342
- JP-A- 2000 287 676
- MORISHITA T: "PRODUCTION OF MENAQUINONES BY LACTIC ACID BACTERIA" JOURNAL OF DAIRY SCIENCE, AMERICAN DAIRY SCIENCE ASSOCIATION, SAVOY, IL, US, vol. 82, no. 9, 1999, pages 1897-1903, XP000983779 ISSN: 0022-0302 cité dans la demande
- DEMAIN A L: "INDUSTRIAL MICROBIOLOGY" SCIENCE (WASHINGTON D C), vol. 214, no. 4524, 1981, pages 987-995, XP002440835 ISSN: 0036-8075

## Description

La présente invention se rapporte au domaine des produits alimentaires riches en nutriments, vitamines et/ou oligo-éléments afin d'améliorer la teneur et l'équilibre qualitatif et quantitatif des apports nutritionnels chez l'homme.

Différents procédés industriels utilisant des micro-organismes permettent de produire des métabolites secondaires à grande échelle (Demain AL. et al, Science 1981).

L'invention s'intéresse aux moyens d'enrichir les aliments en vitamine K.

Plus précisément, la présente invention décrit un procédé pour augmenter la quantité de vitamine K2 obtenue en cultivant au moins une souche de bactérie lactique productrice de vitamine K2, dans lequel la culture de ladite souche est réalisée dans des conditions de « resting cells », de sorte que la quantité de vitamine K2 produite par la culture en « resting cells » est supérieure, d'un facteur au moins égal à environ 1,2, à celle obtenue en cultivant ladite souche dans des conditions de fermentation standard.

En outre, la présente invention vise la biomasse obtenue à partir d'une culture de bactérie lactique productrice de vitamine K2 conformément au procédé ci-dessus.

L'invention concerne également un procédé de production de vitamine K2, des procédés de préparation de produits alimentaires, notamment de produits fermentés et/ou de produits laitiers frais, enrichis en vitamine K2, ainsi que les produits alimentaires ainsi obtenus.

La vitamine K est une vitamine liposoluble qui se présente sous deux formes naturelles : la vitamine K1 (ou phylloquinone) et la vitamine K2 (ou ménaquinone)

La vitamine K1 est synthétisée par les végétaux. On la trouve principalement dans les légumes verts (légumes-feuilles) et l'huile de soja.

La vitamine K1 intervient plus directement dans le processus de coagulation du sang.

La vitamine K2, quant à elle, est produite par les bactéries de la flore intestinale. Elle apparaît également en faibles quantités dans certains aliments à la suite d'un processus de fermentation (fromage, produits asiatiques typiques tels que le miso et le natto japonais, à base de soja fermenté, etc.). De nombreuses bactéries sont capables de synthétiser la vitamine K2. Ainsi, outre les bactéries de la flore intestinale et, notamment, les espèces *Escherichia coli, Bacillus subtilis* et *Bacteroides spp.,* on peut citer certaines espèces ou sous-espèces de bactéries lactiques telles que *Lactococcus lactis* spp. *lactis, Lactococcus lactis* spp. *cremoris, Leuconostoc lactis, Leuconostoc mesenteroides* et *Propionibacterium sp.* La quantité de vitamine K2 synthétisée par ces bactéries varie généralement d'environ 29 à 90 µg/l de lait fermenté (Morishita et al., 1999). Il est important de souligner que les mesures de production de vitamine K2 sont le plus souvent réalisées à partir de lyophilisats de culots cellulaires et les résultats de ces mesures révèle une grande hétérogénéité des niveaux de production en fonction des souches testées, pouvant varier du simple à plus du triple (Morishita et al., 1999 ; Parker et al., 2003). En termes d'activité biologique, la vitamine K2 est surtout connue pour son action sur la calcification des tissus mous.

La vitamine K a initialement été décrite pour son rôle essentiel dans le processus de coagulation du sang. Ainsi, de fortes carences en vitamine K entraînent des hémorragies, avec allongement anormal du temps de coagulation, et des ecchymoses. On a longtemps considéré que les carences fortes en vitamine K étaient plutôt rares chez l'adulte, les besoins pouvant être en principe couverts de manière satisfaisante par une alimentation variée et équilibrée et grâce à la production endogène de la vitamine par les bactéries coliques. A cet égard, les personnes à risque sont typiquement :
- les nouveaux-nés, dont les intestins ne possèdent pas à la naissance les bactéries productrices de vitamine K ;
- les personnes dont les fonctions hépatiques, biliaires ou intestinales sont perturbées (maladies hépatiques, mucoviscidose, colites, dysenteries...) ; et
- celles qui prennent des antibiotiques au long cours.

Plus récemment, on a découvert que l'impact de la vitamine K sur la santé humaine ne se limitait pas à son rôle dans les mécanismes de coagulation sanguine. En effet, depuis les années 80, la vitamine K est également reconnue pour son rôle dans le métabolisme osseux (Hart et al., 1984 ; Hart et al., 1985).

Cette vitamine joue le rôle de cofacteur dans une réaction enzymatique conditionnant l'activité de l'ostéocalcine dans le cadre de la régulation de la formation osseuse (Hauschka PV et al., 1989; Ducy P et al., 1996). Son rôle consiste plus précisément à conditionner la carboxylation de l'ostéocalcine, protéine-clé qui régule le processus de formation osseuse. En cas de déficience en vitamine K, cette réaction n'a pas lieu, entraînant l'augmentation du ratio sanguin ostéocalcine décarboxylée sur ostéocalcine carboxylée (Väänanen *et al.,* 1999).

L'évolution démographique des pays occidentaux s'est traduite par un vieillissement progressif des populations, associé en corollaire à une augmentation de la prévalence des pathologies dégénératives, notamment de l'ostéoporose. A ce titre, l'ostéoporose est désormais reconnue comme un problème majeur de santé publique.

Les estimations démographiques établies dans les années 90 ont tiré la sonnette d'alarme en prévoyant une augmentation considérable de l'incidence de cette pathologie dans les 50 ans à venir, notamment chez les seniors. S'est donc rapidement imposée la nécessité et l'urgence d'entreprendre des actions afin de prévenir cette pathologie, jusque-là rarement dépistée et prise en charge tardivement.

Il est désormais reconnu que la prévention de l'ostéoporose doit commencer dès l'enfance, au travers d'une croissance osseuse optimale, et se poursuivre toute la vie par un maintien de la masse osseuse. On sait que les facteurs nutritionnels jouent un rôle important dans le développement et le maintien du patrimoine osseux. Jusqu'à présent, les stratégies nutritionnelles envisagées ou proposées pour prévenir l'ostéoporose reposent essentiellement sur deux facteurs clés qui sont le calcium et la vitamine D. Pourtant, on sait aujourd'hui que d'autres facteurs nutritionnels pourraient présenter un intérêt notable.

De par son rôle majeur dans la formation osseuse, la vitamine K apparaît de plus en plus dans la littérature comme une piste prometteuse pour préserver la santé osseuse de l'homme tout au long de sa vie.

Les apports nutritionnels recommandés en vitamine K chez l'homme (1,5 µg/j/kg de poids) ont été établis en ne prenant en considération que son rôle dans les phénomènes de coagulation. Or, des études récentes suggèrent que ces apports nutritionnels recommandés sont finalement sous-estimés si l'on prend également en compte l'activité de la vitamine K dans le métabolisme osseux (Ronden et al., 1998).

Si les besoins en vitamine K sont encore mal connus, il n'en demeure pas moins que de faibles apports sont associés à une faible masse osseuse et à un risque accru de fractures chez l'adulte (Hart et al., 1985 ; Knapen et al., 1989 ; Szulc et al., 1993 ; Booth et al, 2000). De plus, des études d'intervention chez les femmes ménopausées ont montré que la vitamine K pouvait permettre de diminuer les pertes osseuses pour cette cible (Shiraki et al., 2000 ; Braam et al., 2003). Enfin, des études chez l'animal suggèrent qu'elle pourrait jouer un rôle favorable au cours du pic de masse osseuse et ce, d'autant mieux en cas d'association synergique avec la vitamine D. Cependant, les études reliant clairement la vitamine K et la croissance osseuse n'ont pour l'heure été menées que chez l'animal.

De plus, des études récentes ont permis d'apporter des arguments supplémentaires en faveur de l'impact de la vitamine K sur le métabolisme osseux et, en particulier, sur la constitution et la préservation de la masse osseuse (Booth et al., 2000; Shiraki et al., 2000; Braam et al., 2003; Hirano et Ishi, 2002).

Contrairement à l'adulte, peu de données sont encore disponibles en ce qui concerne les effets bénéfiques de la vitamine K sur le métabolisme osseux de l'enfant. On sait seulement qu'il est essentiel d'optimiser la masse osseuse pendant la période dé croissance, afin de constituer un réservoir osseux maximal et de protéger l'adulte contre le risque d'ostéoporose à venir.

En tout état de cause, il ressort de l'ensemble des données disponibles à ce jour que l'amélioration de la teneur en vitamine K des produits alimentaires est une piste particulièrement intéressante et prometteuse pour permettre à l'individu de construire et maintenir une bonne constitution osseuse.

Dans ce contexte, il existe déjà des produits industriels sur le marché de l'alimentaire contenant une quantité notable de vitamine K. On peut notamment citer certains produits laitiers renfermant des bactéries lactiques, tels que les « Petits Gervais aux Fruits» commercialisés en France par la Demanderesse. On notera néanmoins que, d'une part, la teneur de ces produits en vitamine K dépend généralement du type de ferments utilisés et, d'autre part, les souches de *Lactococcus lactis* classiquement utilisées dans les produits laitiers ne produisent pas une quantité suffisante de vitamine K pour véritablement combler les besoins de la population, voire pour aider à pallier d'éventuelles carences en vitamine K.

Il existe donc un besoin dans la technique actuelle pour des produits alimentaires, notamment des produits fermentés et/ou des produits laitiers frais, qui renferment de la vitamine K en quantités suffisantes pour contribuer à satisfaire les besoins et, si nécessaire, à combler les carences, tant chez l'enfant et l'adolescent, que chez l'adulte et la personne âgée.

Dans ce qui suit, les termes « vitamine K2 » et « vitamine K » sont utilisés indifféremment pour désigner la vitamine K2.

La présente invention vise donc à répondre à ce besoin en proposant pour la première fois de préparer des produits alimentaires, tels que des produits fermentés et/ou des produits laitiers frais, dans lesquels des ferments capables de produire la vitamine K sont mis en oeuvre dans des conditions qui favorisent de manière tout à fait sensible la production de vitamine K par rapport aux conditions de production classiques.

En outre, au cours de leurs travaux, les Inventeurs ont obtenu de nouveaux variants naturels de souches naturelles de bactéries lactiques, qui produisent des quantités de vitamine K significativement supérieures à celles produites par les souches naturelles desquelles ils dérivent (voir la partie « Exemples » ci-dessous). Ainsi pourra-t-on avantageusement utiliser ces variants « surproducteurs » de vitamine K dans les conditions de mise en oeuvre particulièrement favorables à la production de vitamine K qui font l'objet de la présente invention.

Selon un premier aspect, la présente invention concerne un procédé pour augmenter la quantité de vitamine K2 obtenue en cultivant au moins une souche de bactérie lactique productrice de vitamine K2, dans lequel la culture de ladite souche est réalisée dans des conditions de « resting cells », ledit procédé comprenant au moins :
a) la préculture de ladite souche dans des conditions de respiration, dans un milieu de préculture approprié contenant au moins une porphyrine à une concentration finale d'au moins 0,5µg/mL;
b) l'ensemencement d'un milieu de culture approprié contenant au moins 0,5% de matière grasse avec une quantité de cellules bactériennes vivantes allant de 10⁸ à 10¹¹ ufc/ml environ ; et
c) la fermentation du milieu ainsi ensemencé pendant une période allant de 4 à 48 heures environ, de préférence allant de 8 à 48 heures environ, à une température allant de 4 à 50°C environ, de préférence allant de 4 à 40°C environ, de sorte qu'à l'issue de l'étape c), la quantité de vitamine K2 produite par la culture en « resting cells » est supérieure, d'un facteur au moins égal à environ 1,2, à celle obtenue en cultivant ladite souche dans des conditions de fermentation standard.

L'expression « culture en « resting cells » ou culture dans des conditions de « resting cells » » fait partie du langage commun dans le domaine technique de la présente invention. La notion de « resting cells » est donc parfaitement claire pour l'homme du métier. En France, les techniciens du domaine comprennent et utilisent couramment les termes anglais « resting cells », qui ne nécessitent donc pas d'être traduits. On précise à toutes fins utiles qu'une traduction française appropriée mais peu usitée des termes « resting cells » est « cellules non proliférantes ».

Les « conditions de fermentation standard » sont, comme leur nom l'indique, tout à fait classiques et bien connues de l'homme du métier (on parle également de « conditions de laboratoire »). Les « conditions de fermentation standard » préférées au sens de la présente invention sont les suivantes : une préculture de la souche est réalisée sur milieu commercial M17 (Difco™ M17 Agar) ou sur un milieu équivalent, additionné de 20µl/ml d'une solution d'hémine à 0,5mg/ml dans de la soude 0,1M. Pour la culture qui suit, l'ensemencement est réalisé à 1% à l'aide de la préculture. La température d'incubation est de 30°C environ. L'aération est assurée par simple agitation. On retiendra que les conditions de fermentation peuvent être modifiées en cas de besoin par l'homme du métier, sur la base de ses connaissances générales et, éventuellement, après des expériences de mises au point de routine. Toutefois, on veillera à systématiquement préserver les trois critères essentiels suivants : (i) le milieu de culture est un milieu approprié pour cultiver les souches de bactéries lactiques, notamment les souches de *Lactococcus spp. ;* (ii) au moins un composé contenant un noyau hémique (par exemple, de l'hémine, de la catalase ou des dérivés de chlorophylle) est ajouté dans le milieu de préculture et/ou de culture (de préférence, à la fois dans le milieu de préculture et dans le milieu de culture) ; et (iii) la préculture et/ou la culture (de préférence, la préculture seulement) est(sont) mise(s) en oeuvre sous agitation.

Des modes de réalisation particuliers du procédé selon l'invention sont tels que :
- dans l'étape b), l'ensemencement du milieu de culture est effectué avec une quantité de cellules bactériennes vivantes allant de 5.10⁸ à 10¹⁰ ufc/ml environ et allant plus particulièrement encore de 2.10⁹ à 6.10⁹ ufc/ml environ ;
- dans l'étape c), la fermentation du milieu est effectuée dans les conditions standard pendant une période allant de 12 à 36 heures environ, préférentiellement de 15 à 24 heures environ ;
- dans l'étape c), la fermentation du milieu est menée dans les conditions standard à une température allant de 15 à 35°C environ, de préférence de 20 à 30°C environ.

De préférence, à l'issue de l'étape c), la quantité de vitamine K2 produite par la culture en « resting cells » est supérieure, d'un facteur au moins égal à environ 1,5, à celle obtenue en cultivant ladite souche dans des conditions de fermentation standard. Ce facteur est de manière encore préférée au moins égal à environ 1,7, plus préférentiellement au moins égal à environ 1,8, plus préférentiellement encore au moins égal à environ 1,9. Des valeurs de ce facteur encore davantage préférées sont d'environ 2 ; 2,2 ; 2,4 ; 2,5 ; 2,7 ; 2,8 ; 2,9 ; 3.

On cherche ainsi de préférence à atteindre, grâce aux moyens de l'invention, des niveaux de production de vitamine K2 de l'ordre de 30 µg de vitamine K2 pour 100g de lait fermenté dans des conditions de fermentation standard. Mieux, les niveaux de production peuvent atteindre environ 40 µg de vitamine / 100g de lait fermenté, et, de manière encore préférée, ils peuvent se situer aux environs de, voire dépasser, environ 45 ou 50 µg de vitamine K2 / 100g de lait fermenté. Ainsi, des niveaux de production d'environ 55, 60, 65, 70, 75 µg de vitamine K2 / 100g de lait fermenté, et plus encore, sont tout particulièrement préférés.

Selon un mode de réalisation, la souche de bactérie lactique productrice de vitamine K2, mise en oeuvre dans le cadre du procédé de l'invention est choisie parmi les genres *Lactococcus, Leuconostoc, Enterococcus* et *Propionibacterium.* En particulier, la souche de bactérie lactique utilisée est choisie parmi les espèces *Lactococcus lactis, Leuconostoc lactis, Leuconostoc pseudomesenteroides, Leuconostoc mesenteroides, Leuconostoc dextranicum, Enterococcus faecium, Propionibacterium sp.* De manière avantageuse, la souche de bactérie lactique est choisie parmi les variants naturels de *Lactococcus lactis subsp. cremoris* producteurs de vitamine K2 qui ont été obtenus par les Inventeurs dans le cadre des travaux rapportés dans les Exemples ci-dessous : le variant I-3557 déposé à la Collection Nationale de Culture des Microorganismes (CNCM, Institut Pasteur, 25 rue du Docteur Roux, 75724 Paris cedex 15, France) le 20/01/2006, le variant I-3558 déposé à la CNCM le 20/01/2006 et le variant I-3626 déposé à la CNCM le 19/06/2006.

Le terme « variants » couvre ici :
- les variants naturels, c'est-à-dire obtenus spontanément à partir d'une souche de bactérie lactique de référence sous l'effet d'une pression de sélection ; les variants naturels ne subissent donc aucune manipulation génétique, mais sont obtenus principalement par mutation et sélection à partir de la souche de référence ; et
- les mutants comprenant une ou plusieurs mutations dans leur génome, qui ont été induites par génie génétique, c'est-à-dire par des techniques de mutagénèse. dirigée, notamment par transformation génétique à l'aide de vecteurs, appliquées à la souche de référence.
   En tous les cas, les « variants » sont, au sens de l'invention, des souches capables de produire de la vitamine K2. Avantageusement, si le niveau de production est d'au moins 5,5 µg environ de vitamine K2 pour 100g de lait fermenté dans des conditions de fermentation standard (également appelées « conditions de laboratoire »), on pourra parler de variant « surproducteur » de vitamine K2.

On notera que, dans certains pays (notamment en Europe), des précautions doivent être prises par les industriels de l'alimentaire lorsqu'ils développent des produits destinés à l'alimentation humaine et/ou animale, dans lesquels sont incorporés des microorganismes, plus particulièrement des microorganismes vivants. En effet, les organismes (ici, microorganismes) génétiquement modifiés (OGM ou mutants) peuvent susciter un sentiment de crainte et d'appréhension chez les consommateurs. Cette image négative dont souffrent les OGM dans certains pays est telle que le public a tendance à « boycotter » les aliments contenant des OGM. Aussi, dans un contexte où les consommateurs exigent toujours plus de transparence aux niveaux du contenu des produits alimentaires qui leur sont proposés et de l'origine des ingrédients que ces produits contiennent, les industriels peuvent être amenés à proposer des produits quasi-exclusivement, voire exclusivement, sans OGM. Dans le contexte de la présente invention, il peut donc être avantageux que les produits alimentaires issus de l'industrie et contenant des microorganismes soient préparés en utilisant exclusivement des souches naturelles ou des variants naturels de souches naturelles.

Dans l'étape b), le milieu de culture approprié contient au moins environ 0,5%, plus préférentiellement au moins environ 1,5%, plus préférentiellement encore au moins environ 3,5% de matière grasse. Un tel milieu peut par exemple être à base de crème laitière ou de jus de soja. Il peut aussi s'agir d'un lait ou d'un lait dont le pouvoir tampon a été augmenté. Bien évidemment, des combinaisons de ces différents milieux peuvent également être envisagées. A titre d'exemple de « lait au pouvoir tampon augmenté », on citera notamment un lait additionné de β-glycérolphosphate et/ou citrate et/ou protéines de lait et/ou n'importe quel ingrédient alimentaire approprié doté d'un pouvoir tampon.

Par exemple, un lait demi-écrémé contient typiquement environ 1,5% environ de matière grasse ; un lait entier en contient généralement 3,5% environ.

Le procédé objet de la présente invention comprend une étape préliminaire à l'étape b), qui consiste à précultiver la souche dans des conditions de respiration, dans un milieu de préculture approprié contenant au moins une porphyrine à une concentration finale d'au moins environ 0,5 µg/ml. Des concentrations et/ou gammes de concentrations de porphyrine encore davantage préférées sont d'au moins 1 µg/ml environ, mieux d'au moins 5 µg/ml environ, et mieux encore d'au moins 10 µg/ml environ.

WO 2000/05342 décrit un procédé dans lequel des souches bactériennes sont cultivées en présence de porphyrine. Toutefois, cette demande ne suggère pas que cette étape permet d'augmenter la production de vitamine K2 chez des bactéries lactiques.

Avantageusement, la préculture est incubée à une température allant de 4 à 40°C environ, de préférence à une température allant de 18 à 35°C environ, avec une valeur préférée à 30°C environ.

Le temps d'incubation de la préculture peut varier en fonction des souches et des autres conditions de mise en oeuvre. Il est de préférence d'au moins 8 heures environ, préférentiellement d'au moins 12 heures environ, plus préférentiellement encore d'au moins 16 heures environ.

L'oxygénation de la préculture peut être réalisée par agitation ou aération.

Une étape intermédiaire peut en outre être mise en oeuvre entre l'étape préliminaire de préculture et l'étape b) du procédé objet de la présente invention. Cette étape intermédiaire consiste à concentrer la biomasse obtenue à l'issue de la préculture, par exemple par centrifugation de la préculture suivie de la récupération du culot bactérien.

On notera que, compte tenu des applications de l'objet de la présente invention dans le domaine alimentaire, on mettra de préférence en oeuvre des conditions qui sont tout à la fois (i) applicables et exploitables à l'échelle industrielle (en termes de faisabilité, rendement, coût, équipement, etc.), et (ii) appropriées à des produits alimentaires (en termes de propriétés physiques et organoleptiques des produits finis (goût, odeur, texture, aspect, etc.)).

Un deuxième aspect de la présente invention a trait à la biomasse enrichie susceptible d'être obtenue par culture d'au moins une souche de bactérie lactique productrice de vitamine K2 dans des conditions de « resting cells » conformément au procédé précédemment décrit.

Dans un troisième aspect de la présente invention, on utilise la biomasse susmentionnée pour préparer un produit alimentaire enrichi en vitamine K2.

Un quatrième aspect de la présente invention concerne un procédé pour produire de la vitamine K2, qui comprend au moins :
a) la mise en oeuvre du procédé pour augmenter la quantité de vitamine K2 obtenue en cultivant au moins une souche de bactérie lactique productrice de vitamine K2, conformément à la description précédente ; et
b) la récupération de la vitamine K2 ainsi produite.

Selon un cinquième aspect, la présente invention vise des procédés pour préparer un produit alimentaire enrichi en vitamine K2, ou pour enrichir un produit alimentaire en vitamine K2.

Selon un premier mode de réalisation, un tel procédé comprend au moins :
a) la production de vitamine K2 selon le procédé objet du quatrième aspect de l'invention ;
b) l'ajout de la vitamine K2 ainsi produite audit produit alimentaire, ou à une préparation intermédiaire de celui-ci ; et
c) l'obtention dudit produit alimentaire enrichi en vitamine K2.

Selon un deuxième mode de réalisation, un procédé pour préparer un produit alimentaire enrichi en vitamine K2 comprend au moins :
a) la culture d'au moins une souche de bactérie lactique productrice de vitamine K2 dans des conditions de « resting cells » conformément au procédé pour augmenter la quantité de vitamine K2 obtenue à partir d'une culture de cette souche (premier aspect de l'invention) ;
b) l'ajout de la biomasse obtenue à partir de la culture sous a), audit produit alimentaire ou à une préparation intermédiaire de celui-ci ; et
c) l'obtention dudit produit alimentaire enrichi en vitamine K2.

Alternativement, on peut prévoir de réaliser simultanément les étapes a) et b) ci-dessus :
a) la culture d'au moins une souche de bactérie lactique productrice de vitamine K2 dans des conditions de « resting cells » conformément au procédé pour augmenter la quantité de vitamine K2 obtenue à partir d'une culture de cette souche (premier aspect de l'invention), dans ledit produit alimentaire ou dans une préparation intermédiaire de celui-ci ; et
b) l'obtention du produit alimentaire enrichi en vitamine K2.

Dans ce cas, la ou les souches peu(ven)t notamment être mise(s) en oeuvre en utilisant des concentrats de bactéries précultivées sur place (sur le site de production des produits alimentaires), ou en utilisant des bactéries précultivées par un fournisseur de ferments, puis conditionnées et expédiées vers le(s) site(s) de production des produits alimentaires. Les fournisseurs peuvent conditionner les bactéries à l'état frais ou congelé ; alternativement, les bactéries peuvent être séchées ou lyophilisées. Les bactéries sont, dans tous les cas, ajoutées à la masse laitière de manière tout à fait classique (comme n'importe quel autre ferment lactique connu). Pour l'étape suivante de culture dans des conditions favorables à la production de vitamine K2, on applique les conditions de mise en oeuvre qui font l'un des objets de la présente invention.

Encore un autre mode de réalisation d'un procédé pour enrichir un produit alimentaire en vitamine K2 comprend au moins :
a) l'ajout de la biomasse conforme à la présente invention, audit produit alimentaire ou à une préparation intermédiaire de celui-ci ; et
b) l'obtention dudit produit alimentaire enrichi en vitamine K2.

Typiquement, on utilise la biomasse comme un ferment lactique classique.

Un sixième aspect de la présente invention est relatif à un produit alimentaire enrichi en vitamine K2 susceptible d'être obtenu par la mise en oeuvre d'un procédé tel que susdécrit.

Alternativement, un produit alimentaire enrichi en vitamine K2 selon la présente invention contient la biomasse décrite ci-dessus.

L'invention s'intéresse aux produits alimentaires pour l'homme et/ou l'animal, avec une préférence pour les produits destinés à l'alimentation humaine. Avantageusement, un tel produit alimentaire enrichi en vitamine K2 renforce la solidité des os de la personne qui le consomme. Cette personne est, de manière préférée, un enfant.

De préférence, un produit alimentaire au sens de l'invention est choisi parmi les produits fermentés, les produits laitiers frais fermentés ou non, les produits à base de jus d'origine végétale (fruits, légumes, céréales, soja, etc.) fermentés ou non, et leurs combinaisons. De manière plus particulièrement préférée, un produit alimentaire au sens de l'invention est un produit fermenté et/ou un produit laitier frais.

Dans le contexte de l'invention, les «produits laitiers frais » désignent plus particulièrement des produits laitiers frais et fermentés, prêts à la consommation humaine, c'est-à-dire des aliments laitiers frais et fermentés. Dans la présente demande, sont plus particulièrement visés les laits fermentés et yoghourts. Lesdits aliments laitiers frais et fermentés peuvent alternativement être des fromages blancs ou des petits-suisses.

On donne aux termes « laits fermentés » et « yoghourts » leurs significations usuelles dans le domaine de l'industrie laitière, c'est-à-dire des produits qui sont destinés à la consommation humaine et qui sont issus de la fermentation lactique acidifiante d'un substrat laitier. Ces produits peuvent contenir des ingrédients secondaires tels que fruits, végétaux, sucre, etc. On peut, par exemple, se reporter au Décret français n° 88-1203 du 30 décembre 1988 relatif aux laits fermentés et au yaourt ou yoghourt, publié au Journal Officiel de la République Française du 31 décembre 1988.

On peut également se reporter au « Codex Alimentarius » (préparé par la Commission du Codex Alimentarius sous l'égide de la FAO et de l'OMS, et publié par la Division Information de la FAO, disponible en ligne sur http://www.codexalimentarius.net; cf. plus particulièrement le volume 12 du Codex Alimentarius « Normes Codex pour le lait et les produits laitiers », et la norme « CODEX STAN A -1 1(a)-1975 »).

L'expression « lait fermenté » est ainsi réservée dans la présente demande au produit laitier préparé avec un substrat laitier qui a subi un traitement au moins équivalent à la pasteurisation, ensemencé avec des microorganismes appartenant à l'espèce ou aux espèces caractéristique(s) de chaque produit. Un « lait fermenté » n'a subi aucun traitement permettant de soustraire un élément constitutif du substrat laitier mis en oeuvre et n'a notamment pas subi un égouttage du coagulum. La coagulation des « laits fermentés » ne doit pas être obtenue par d'autres moyens que ceux qui résultent de l'activité des microorganismes utilisés.

Le terme « yoghourt » est quant à lui réservé au lait fermenté obtenu, selon les usages locaux et constants, par le développement des bactéries lactiques thermophiles spécifiques dites *Lactobacillus bulgaricus* et *Streptococcus thermophilus,* qui doivent se retrouver vivantes dans le produit fini, à raison d'au moins 10 millions de bactéries par gramme rapportées à la partie lactée.

Dans certains pays, la réglementation autorise l'ajout d'autres bactéries lactiques dans la production de yoghourt, et notamment l'utilisation additionnelle de souches de *Bifidobacterium* et/ou *Lactobacillus acidophilus* et/ou *Lactobacillus casei.* Ces souches lactiques additionnelles sont destinées à conférer au produit fini diverses propriétés, telles que celle de favoriser l'équilibre de la flore intestinale ou de moduler le système immunitaire.

Dans la pratique, l'expression « lait fermenté » est donc généralement utilisée pour désigner les laits fermentés autres que les yoghourts. Elle peut d'ailleurs prendre, selon les pays, des noms aussi divers que, par exemple, « Kefir », « Kumiss », « Lassi », « Dahi », « Leben », « Filmjôlk », « Villi », « Acidophilus milk ».

S'agissant des laits fermentés, la quantité d'acide lactique libre contenue dans le substrat laitier fermenté ne doit pas être inférieure à 0,6 g pour 100 g lors de la vente au consommateur, et la teneur en matière protéique apportée à la partie lactée ne doit pas être inférieure à celle d'un lait normal.

Enfin, la dénomination « fromage blanc » ou « petit-suisse » est, dans la présente demande, réservée à un fromage non affiné, non salé, qui a subi une fermentation par des bactéries lactiques uniquement (et aucune autre fermentation que la fermentation lactique). La teneur en matière sèche des fromages blancs peut être abaissée jusqu'à 15 g ou 10 g pour 100 g de fromage blanc, selon que leur teneur en matières grasses est supérieure à 20 g, ou au plus égale à 20 g pour 100 g de fromage blanc, après complète dessication. La teneur en matière sèche d'un fromage blanc est comprise entre 13 et 20 %. La teneur en matière sèche d'un petit-suisse quant à elle n'est pas inférieure à 23 g pour 100 g de petit-suisse. Elle est généralement comprise entre 25 et 30 %. Les fromages blancs et petits-suisses sont généralement regroupés sous la dénomination « fromages frais », utilisée de manière classique dans le domaine technique de la présente invention.

Les figures suivantes illustrent la présente invention, sans néanmoins en limiter ni l'objet ni la portée.
- Figure 1 : Histogramme illustrant l'influence de la concentration en matière grasse des laits sur la production de vitamine K2 par les bactéries lactiques. Souches 1 et 2: exemples de souches naturelles de *Lactococcus lactis* ssp. *cremoris.*
- Figure 2 : Exemples de cinétique de production de la vitamine K2 sur lait entier et de cinétique d'acidification (cadre supérieur gauche) pour une souche naturelle de bactérie lactique (souche n° 1).
- Figure 3 : Histogramme présentant la production de vitamine K2 par des cultures en « resting cells » à différentes températures. Témoin : culture dans des conditions de croissance standard.
- Figure 4: Histogramme présentant l'influence des conditions de préculture sur la production de vitamine K2 selon les souches.

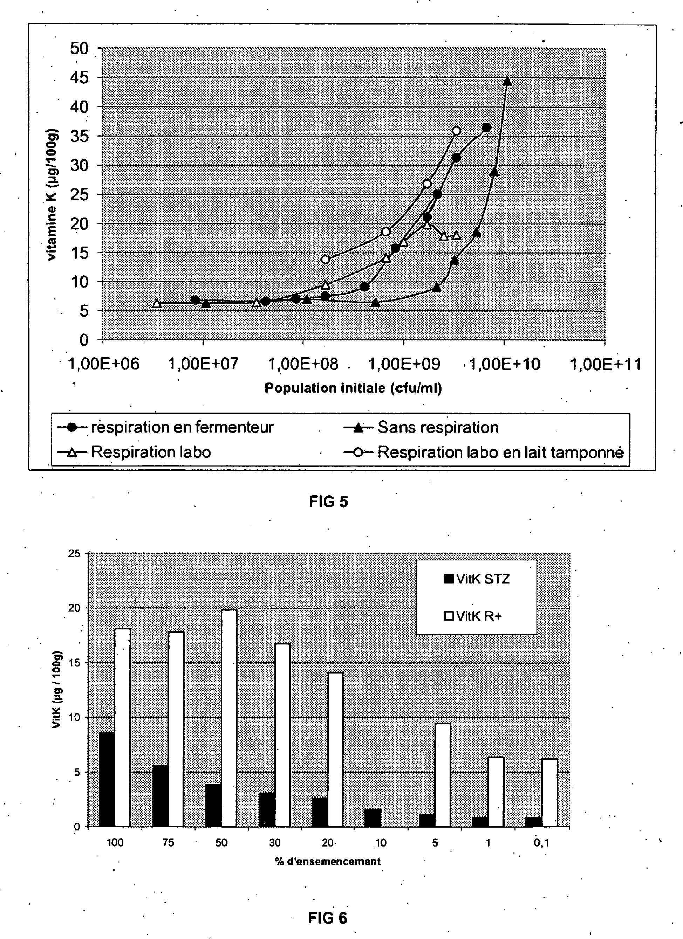

- Figure 5 : Graphique illustrant l'influence de la population bactérienne initiale du variant naturel I-3558 sur la production de vitamine K2 pendant la phase de «resting cells », réalisée en lait classique ou en lait tamponné. « Sans respiration » : préculture classique puis culture en conditions de « resting cells» en lait entier. « Respiration labo » : préculture dans des conditions de respiration réalisée en tube puis culture en conditions de « resting cells» en lait entier. « Respiration labo en lait tamponné » : préculture dans des conditions de respiration puis culture en conditions de « resting cells » en lait tamponné par le β-glycérolphosphate. « Respiration en fermenteur » : préculture dans des conditions de respiration réalisée en fermenteur suivie d'une culture en conditions de « resting cells » en lait classique.
- Figure 6: Histogramme présentant l'influence de la viabilité bactérienne du variant naturel 1-3558 sur la production de vitamine K2 pendant la phase de « resting cells ». VitK STZ : préculture en conditions de respiration traitée à la streptozotocine puis culture en conditions de « resting cells » en lait entier additionné d'érythromycine. VitK R+ : préculture dans des conditions de respiration réalisée en tube puis culture en conditions de « resting cells » en lait entier.

Il est clair que la présente invention ne se limite pas à la seule description ci-dessus. D'autres modes de réalisation et avantages de l'invention pourront ressortir à la lecture des exemples ci-dessous, fournis à titre purement illustratif.

### EXEMPLES

### PARTIE A : OBTENTION DE VARIANTS NATURELS DE SOUCHES NATURELLES DE BACTERIES LACTIQUES CAPABLES DE PRODUIRE DES QUANTITES AVANTAGEUSES DE VITAMINE K

A titre de remarques préliminaires, il est à noter que les protocoles d'obtention de variants naturels décrits ci-dessous sont applicables à n'importe quel type de souche de bactérie lactique de départ. En fonction des souches de départ que l'homme du métier utilisera, il pourra éventuellement être amené, pour des raisons essentiellement pratiques, à modifier certaines des conditions expérimentales mises au point par les Inventeurs. En tout état de cause, les modifications que l'homme du métier sera susceptible d'apporter aux procédures ci-dessous seront mineures et nécessiteront uniquement de simples manipulations de routine n'impliquant aucune activité inventive.

### A-I- Obtention et utilisation de variants naturels résistants à la bacitracine

Bien qu'une exposition à des agents tels que la bacitracine ou le péroxyde soit connu pour permettre de sélectionner des souches bactériennes qui présentent une résistance accrue à ces agents, il n'a jamais été établi de lien dans la littérature entre la résistance à la bacitracine ou au péroxyde et les niveaux de production de vitamine K2 par les bactéries.

Dans le cadre de leurs travaux, les Inventeurs ont découvert de manière tout à fait inattendue que les bactéries étaient capables de développer un mécanisme original de résistance à certains agents tels que la bacitracine ou le péroxyde, impliquant une augmentation de la production de vitamine K2. Les Inventeurs ont envisagé de mettre à profit cette découverte aux fins de l'obtention, en utilisant la bacitracine ou le péroxyde, par exemple, comme agent de sélection, de variants naturels de souches de bactéries lactiques (notamment *Lactococcus lactis)* capables de surproduire la vitamine K2.

### A-I-1- Protocole d'obtention de variants résistants à la bacitracine

Une préculture a été réalisée à partir d'un cristal d'une souche naturelle de *Lactococcus lactis* en présence de 2 mL de milieu de culture commercial classique M17 (milieu M17 Agar, Difco™) additionné de lactose à 5g/l (ci-après, milieu M17 Lac) et d'hémine (20 µL/mL) (ci-après, milieu M17 Lac + hémine). L'incubation a été effectuée sous agitation à 30°C.

La préculture a servi à ensemencer 2 mL de M17 Lac + hémine supplémenté en bacitracine (4µg/mL). Le taux d'ensemencement était de 1 %. La culture a ensuite été incubée pendant 48H sous agitation à 30°C.

Puis, 100 µL de cette suspension ont été déposés sur une gélose de M17 Lac. Un disque de papier imbibé avec 2,5 mg de bacitracine a été déposé au centre de la boîte. La gélose a été incubée 48H à 30°C. Les clones proches du disque de papier ont été cultivés en présence de bacitracine (4µg/mL) dans 2mL de M17 Lac + hémine. L'incubation a duré 24H sous agitation à 30°C.

Les cellules ont été isolées sur gélose M17 Lac en présence de bacitracine (2µg/mL) après une incubation de 48H à 30°C. Les clones isolés ont été cultivés en M17 Lac + hémine, puis incubés pendant 24H sous agitation à 30°C. Cette suspension a servi à l'élaboration du stock congelé.

Ces expériences ont permis aux Inventeurs de sélectionner le variant naturel *Lactococcus lactis subsp. cremoris* I-3557 déposé à la CNCM le 20/01/2006.

### A-I-2- Protocole de réalisation d'un exemple de produit laitier avec le variant. « bacitracine

Une préculture a été réalisée à partir d'un cristal de la souche dans 2 mL de M17 Lac.

La préculture a servi à ensemencer, à 1%, 50 mL de lait entier UHT qui ont été incubés à 30°C pendant 24H.

Le Tableau 1 ci-dessous donne le résultat du dosage de vitamine K2 exprimé en µg Equivalent MK-4/100g de produit, pour le variant bacitracine-résistant et la souche correspondante sauvage.

**Tableau I**

| **Souche** | I-3557 | sauvage |
|---|---|---|
| **Vitamine K (en µg/100g)** | 8,90 | 3,32 |

Le variant bacitracine-résistant surproduit donc, à raison d'un facteur 3, la vitamine K lorsqu'on le compare à la souche sauvage de départ.

### A-II- Obtention et utilisation de variants naturels résistants au peroxyde

La respiration de *Lactococcus lactis* a été mise en évidence assez récemment (Duwat *et al.,* 2001). Le séquençage du génome d'une souche de *L. lactis* (IL1403) a confirmé la présence des gènes codant les fonctions nécessaires à la respiration aérobie (Bolotin *et al.,* 2001). *L. lactis* possède en effet les opérons *men* et *cytABCD* codant les protéines nécessaires à la synthèse de ménaquinone et à la biogenèse du cytochrome D. Cette espèce possède également les trois gènes impliqués dans les dernières étapes de la synthèse d'hème *(hemH, hemK* et *hemN,* qui sont requis dans l'oxydation de la porphyrine pour l'attachement du fer à l'hème), mais ne possède pas les gènes impliqués dans les premières étapes de ce processus. Toutefois, *L. lactis* est capable de réaliser une phosphorylation oxydative en présence de protoporphyrinogène.

On a également montré que *L*. *lactis* pouvait respirer en présence d'oxygène et d'hème dans le milieu de culture. Cette respiration permet aux cellules d'atteindre une biomasse plus importante et le pH final observé est plus élevé que celui habituellement obtenu. Des cultures en présence d'oxygène et/ou d'hème permettent d'obtenir des courbes de croissances comparables pendant approximativement les 6 ou 7 premières heures de fermentation. Ensuite, la consommation de glucose diminue dans le cas des cultures en présence d'oxygène et d'hème, et la production de lactate est alors moindre. Cela traduit un « shift » de métabolisme qui se produit assez tardivement au cours de la culture. La respiration de *L*. *lactis* se fait donc vers la fin de la phase exponentielle de croissance (Duwat *et al.,* 2001).

Le rôle de la respiration de *L. lactis* n'est pas encore connu, pas plus que le rôle que peut tenir la vitamine K2 chez cette espèce à métabolisme plutôt fermentaire. Les Inventeurs ont d'ailleurs constaté que la vitamine K2 était produite par des souches de *L. lactis* alors que la respiration n'était pas induite dans les conditions testées (pas d'hème dans le milieu et pas d'agitation permettant une bonne oxygénation du milieu).

Dans le cytoplasme, les protéines ne présentent que peu de ponts disulfures contrairement aux protéines extracellulaires. Il existe un système enzymatique largement répandu qui permet de limiter le nombre de ponts disulfures. Les liaisons S-S sont réduites en fonction SH par l'intermédiaire d'une enzyme, la thioredoxine. Cette enzyme est régénérée par la thioredoxine réductase. Vido *et al* (2005) ont créé par génie génétique un mutant *trxB1* de *L*. *lactis.* Le gène *trxB1* code pour la thioredoxine réductase. L'étude par électrophorèse bi-dimensionnelle des protéines synthétisées par ce mutant a montré qu'il surproduisait certaines des enzymes de la voie de synthèse de la vitamine K2, à savoir les enzymes MenB et MenD.

Au vu de ces données ainsi que d'après des observations personnelles, les Inventeurs ont supposé que l'une des voies possibles pour améliorer la production de vitamine K2 par *L. lactis* pourrait être d'induire la respiration. Une autre piste pourrait être de tenter de mobiliser la vitamine K2 pour répondre à un stress oxydatif.

Les Inventeurs ont donc cherché à obtenir des variants naturels résistants à un stress oxydatif.

### A-II-1-Protocole d'obtention de variants résistants à un stress oxydatif

Le péroxyde a été choisi comme exemple d'agent oxydant utilisable. Bien entendu, d'autres agents oxydants tels que les ions hyperchloriques, les ions ferreux, la ménadione, le paraquat, l'oxygène ou n'importe quel autre composé oxydant approprié, pourraient être utilisés dans des conditions similaires.

Après une préculture sur milieu M17 Lac, les souches naturelles de départ ont été repiquées sur le même milieu contenant des concentrations croissantes de péroxyde (par exemple, une gamme allant de au moins 20 à au moins 25, 27, 28,5 mg/l environ). Les cultures ont été incubées à 30°C. Après 24h, les premiers tubes de la gamme de concentration ne présentant pas de croissance ont été remis à incuber pendant 24h supplémentaires. Les clones ont ensuite été isolés par épuisement sur milieu gélosé. Un clone a été sélectionné pour une concentration de péroxyde de 27 mg/l. Les Inventeurs ont noté qu'au-delà d'une concentration de péroxyde de 28,5 mg/l, il n'y avait pas de croissance.

Ces expériences ont ainsi permis aux Inventeurs de sélectionner le variant naturel *Lactococcus lactis subsp. cremoris* I-3558 déposé à la CNCM le 20/01/2006.

### A-II-2- Protocole de réalisation d'un exemple de produit laitier avec le variant « péroxyde »

Le clone sélectionné a été mis en croissance dans du lait entier pendant 24h. Des échantillons ont ensuite été prélevés et congelés à - 80°C pour un dosage ultérieur de vitamine K.

Le Tableau Il ci-dessous indique la quantité de vitamine K2 produite par le variant résistant au péroxyde, comparée à la quantité produite par la souche de départ (quantités exprimées en µg Equivalent MK-4/100g de lait fermenté).

**Tableau II**

| **Souche** | **Vitamine K (µg/100g)** |
|---|---|
| Sauvage | 2,92 ± 0,45 |
| I-3558 | 5,94 ± 0,76 |

Comme le montre le Tableau II ci-dessus, le variant produit environ deux fois plus de vitamine K2 que la souche sauvage correspondante.

### A-III- Obtention et utilisation de variants naturels résistants à des analogues structuraux des acides aminés aromatiques

Les acides aminés aromatiques exercent un « feed-back » négatif sur leur propre voie de synthèse, au niveau d'une étape commune avec les voies de synthèse de la vitamine K et des folates. Lorsque ces acides aminés sont présents dans le milieu, ces voies ne sont pas activées. Les Inventeurs ont donc cherché à lever cette régulation négative.

### A-III-1- Protocole d'obtention de variants résistants à des analogues structuraux des acides aminés aromatiques

Une souche naturelle de *L. lactis* a été étalée sur un milieu gélosé chimiquement défini (Cocaign-Bousquet, M., et al., 1995) ne contenant ni tryptophane, ni phénylalanine, ni tyrosine. Les termes "milieu chimiquement défini » sont parfaitement compris de l'homme de l'art. Il s'agit d'un milieu ne contenant que des composés simples et clairement définis (par exemple: vitamine B9, vitamine B12, adénine, tyrosine...), contrairement à un milieu semi-synthétique qui contient des composés complexes (par exemple : extrait de levure, hydrolysat de caséine...).

Un coupon buvard a été disposé au centre des boîtes de pétri et imbibé de 80µl d'une solution contenant 50mM des composés suivants : m-fluorophénylalanine, p-fluorophénylalanine, m-fluorotyrosine et phénylalaminamide. Ces composés sont des analogues structuraux des acides aminés aromatiques. Les boîtes ont été incubées à 30°C. Une zone d'inhibition de croissance est apparue autour des disques. Après 48h, des clones résistants sont apparus dans cette zone. Ces clones ont été remis en croissance sur le milieu chimiquement défini ne contenant pas d'acides aminés aromatiques. Le milieu a été additionné d'une solution contenant les analogues structuraux de ces acides aminés. La concentration finale de chacun de ces composés était de 1mM.

### A-III-2- Protocole de réalisation d'un exemple de produit laitier avec les clones « acides aminés aromatiques »

Les cultures obtenues conformément au paragraphe A-III-1 supra ont servi à ensemencer 50ml de milieu M17 Lac (taux d'ensemencement de 1%) additionné de 1ml d'une solution d'hémine (500mg/l). Les mêmes cultures ont servi à ensemencer un milieu ne contenant pas d'hémine, mais placé sous agitation. Un dernier type de culture a été réalisé. Un milieu M17L a été ensemencé et placé à 30°C sans agitation. Les cultures ont été placées une nuit à 30°C sous agitation (250rpm). Ces cultures ont ensuite été centrifugées 5 min à 6000g. Le surnageant a été retiré et remplacé par 50ml de lait entier. Après 24h, les laits fermentés ont été placés à -80°C en attendant le dosage de la vitamine K2 (voir paragraphe B-VI ci-dessous).

Les expériences de dosage de la vitamine K2 ont permis aux Inventeurs de sélectionner un des clones comme étant un variant naturel surproduisant la vitamine K2 (voir paragraphe B-VI ci-dessous) : il s'agit du variant naturel *Lactococcus lactis subsp. cremoris* I-3626 déposé à la CNCM le 19/06/2006.

### PARTIE B : MISE AU POINT DES CONDITIONS DE MISE EN OEUVRE DES BACTERIES LACTIQUES POUR FAVORISER LA PRODUCTION DE VITAMINE K

### B-I- L'influence de la matière grasse dans le milieu

Lors de dosages effectués sur différents produits, les Inventeurs ont constaté que le produit contenant le plus de vitamine K2 était la crème fermentée. De plus, la vitamine K2 est fortement hydrophobe. Les Inventeurs ont donc émis l'hypothèse que la présence de matière grasse, ou pour le moins, un environnement hydrophobe, pouvait favoriser la production de vitamine K2.

Des fermentations ont donc été réalisées sur des laits contenant différentes concentrations de matière grasse. Les précultures ont été réalisées sur milieu M17 Lac. Les laits ont été ensemencés à 1%. La fermentation a été maintenue à 30°C pendant 24h. Ensuite, des échantillons ont été congelés en attendant un dosage ultérieur.

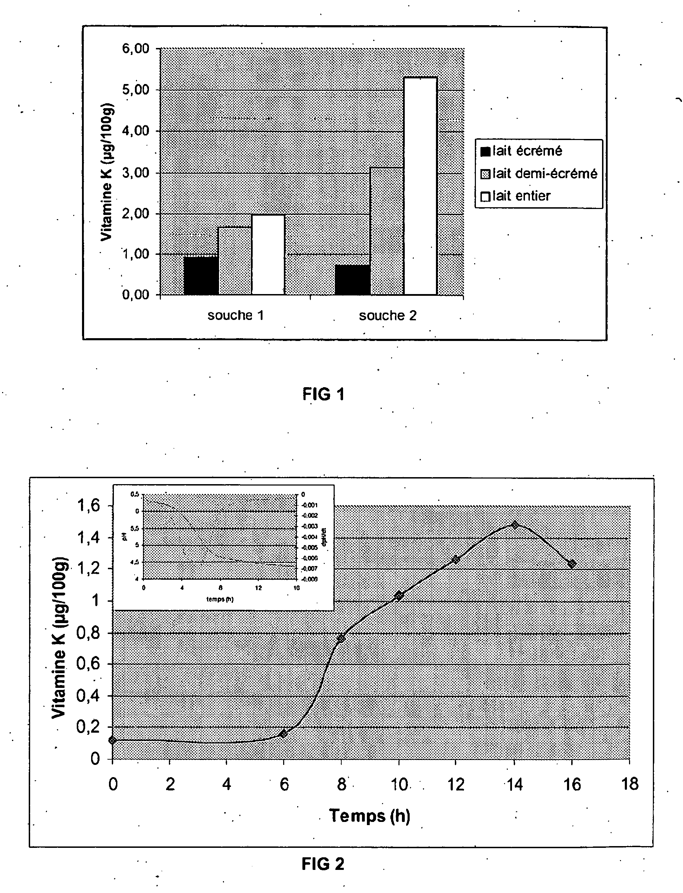

Les résultats sont représentés dans la figure 1, pour deux souches naturelles de *L. lactis subsp. cremoris* (souches n°1 et 2).

Si l'on considère les résultats obtenus avec l'une des deux souches naturelles étudiées à titre d'exemples, l'utilisation d'un lait demi-écrémé (à 1,5% de matière grasse environ) au lieu d'un lait écrémé permettait d'augmenter la production de vitamine K2 d'un facteur 4. Le passage d'un lait demi-écrémé à un lait entier (3,5% de matière grasse environ) permettait de gagner un facteur 2.

Cette tendance était la même quelle que soit la souche naturelle considérée.

Cependant, l'augmentation de la quantité de vitamine K2 produite avec celle de la teneur du milieu en matière grasse semble asymptotique puisque la fermentation d'une crème à 40% de matière grasse environ n'a pas permis d'obtenir des quantités de vitamine K2 supérieures à celles obtenues sur un lait entier (données non montrées).

### B-II- L'influence du taux de croissance des bactéries lactiques

Lors de la croissance en lait d'une souche naturelle de *L. lactis subsp. cremoris* (souche naturelle n°1), les Inventeurs ont réalisé un suivi de la cinétique de la production de vitamine K2 .

Comme le montre la figure 2, la production de vitamine ne démarrait que lorsque la croissance ralentissait. La vitesse de croissance pouvait être appréhendée par le suivi de la cinétique d'acidification. Lorsque la vitesse maximale d'acidification était atteinte, la bactérie entrait en phase de ralentissement.

Ce type de comportement est relativement classique lors de la synthèse de métabolites secondaires. Différents paramètres peuvent être étudiés afin de diminuer le taux de croissance : des conditions physico-chimiques sub-optimales (pH, température...), des composés bactériostatiques (antibiotiques), la culture en « resting cells ». Cette dernière technique consiste à réaliser un ensemencement avec une quantité de cellules correspondant au moins à celle obtenue normalement en fin de fermentation classique. Dans ce cas, il n'y a pas de croissance bactérienne, le taux de croissance est nul.

Les Inventeurs ont ainsi cherché à combiner culture en « resting cells » et effet de la température.

Un lait entier a été ensemencé avec un FED (Ferment à Ensemencement Direct) contenant 10¹¹UFC/g à raison de 10g/l. Le lait a ensuite été mis à incuber à différentes températures.

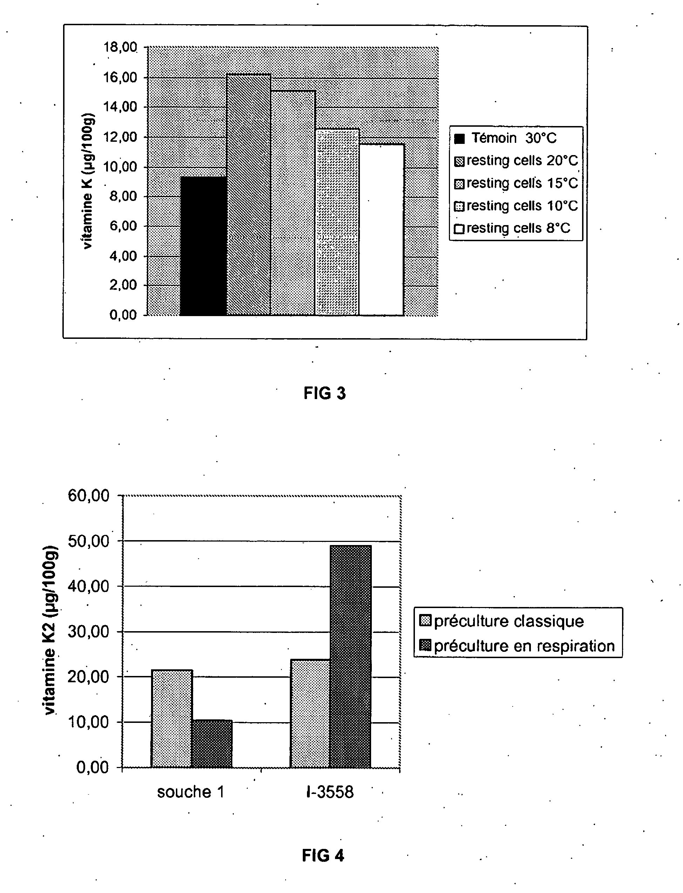

Comme le montre la figure 3, l'abaissement de la température n'avait pas un impact positif sur la production de vitamine K2. En revanche, le fait de cultiver les bactéries en « resting cells » a permis de multiplier la production par un facteur 2 environ: 20µg/100g contre 10µg/100g pour une fermentation classique avec croissance.

### B-III- L'influence de la mise en oeuvre de la préculture en conditions de respiration

La vitamine K2 participe à la chaîne respiratoire. *L. lactis* est capable de respirer mais cette respiration n'intervient qu'en fin de fermentation, c'est-à-dire lorsque le flux métabolique se ralentit. Cette propriété peut être rapprochée de ce qui a été observé lors des cinétiques de production de la vitamine K2.

Il est important de noter que fabriquer entièrement un produit alimentaire en conditions de respiration paraît difficilement réalisable à l'échelle industrielle. En effet, se poseraient alors des problèmes d'aération, d'agitation, de mousse, etc., difficiles à surmonter sans revoir les appareillages et procédés de fabrication usuels, ce qui nécessiterait des investissements très importants et générerait des surcoûts de production inacceptables pour l'industrie agro-alimentaire.

En revanche, le cas échéant, la préculture pourrait être réalisée en conditions de respiration sans que cela pose trop de difficultés aux industriels.

Les Inventeurs ont donc étudié l'impact de la préculture en conditions de respiration sur la production de vitamine K2 par les bactéries lactiques.

Pour ce faire, des précultures ont été réalisées sur milieu M17 Lac additionné de 20µl/ml d'une solution d'hémine à 0,5mg/ml dans de la soude 0,1 M. L'ensemencement a été réalisé à 1% et la température d'incubation était de 30°C. L'aération était assurée par simple agitation.

Dans un premier temps, ces précultures ont permis d'ensemencer du lait entier à 1%, soit une fermentation classique. Dans ces conditions, aucun impact positif sur la production de vitamine K2 n'a été observé (données non montrées).

Ces précultures ont ensuite été utilisées pour réaliser des fermentations en conditions de « resting cells ». Les précultures ont été réalisées comme décrites précédemment et maintenues pendant une nuit. Des échantillons de 50ml ont été centrifugés à 6000g pendant 5 min. Le surnageant a été retiré et remplacé par du lait entier. Le lait a ensuite été incubé à 30°C pendant 24h. Les échantillons ont été congelés à -80°C pour un dosage ultérieur.

Comme le montre la figure 4, le comportement observé était différent selon les souches. La préculture en conditions de respiration n'a pas eu d'impact sur la production de vitamine K2 par la souche naturelle de *L. lactis subsp. cremoris* n°1. En revanche, elle a permis d'augmenter d'un facteur 2, la production de vitamine K2 par le variant naturel I-3558.

La piste de la préculture en conditions de respiration est donc apparue comme intéressante pour certaines souches au moins.
- Toutefois, en pratique, il est important de pouvoir disposer, non pas de précultures fraîches mais de ferments congelés. De plus, afin de réaliser des fermentations en conditions de « resting cells », il convient de disposer de ferments concentrés.

### B-IV- Résultats complémentaires concernant l'effet stimulant de la respiration sur la production de vitamine K2

Ces résultats sont à mettre en relation avec le paragraphe A-III ci-dessus.

Comme indiqué dans le paragraphe A-III-2 supra, la culture du variant naturel I-3626 sélectionné en présence d'analogues structuraux des acides aminés aromatiques a servi à ensemencer 50ml de milieu M17 Lac (taux d'ensemencement de 1%) additionné de 1ml d'une solution d'hémine (500mg/l). La même culture a servi à ensemencer un milieu ne contenant pas d'hémine mais placé sous agitation. Un dernier type de culture a été réalisé. Un milieu M17 Lac a été ensemencé et placé à 30°C sans agitation. Les cultures ont été placées une nuit à 30°C sous agitation (250rpm). Ces cultures ont ensuite été centrifugées 5 min à 6000g. Le surnageant a été retiré et remplacé par 50ml de lait entier. Après 24h, les laits fermentés ont été placés à -80°C en attendant le dosage de vitamine K2.

Le Tableau III ci-dessous donne les résultats de production de la vitamine K2 par le variant naturel I-3626 en fonction des conditions de mise en oeuvre. La quantité de vitamine K2 est exprimée en µg Equivalent MK-4 pour 100g de lait fermenté.

**Tableau III**

| **Type de préculture** | Sans agitation | Avec agitation |
|---|---|---|
| | Sans hémine | Avec hémine |
| **Vitamine K2** | 0,77 | 25,77 |

Ces résultats montrent que la respiration (présence d'hémine sous agitation) a un impact très important sur la production de vitamine K2. Dans ces conditions, la production de vitamine est multipliée par 5. La souche mère produisait 21,5µg/100g après une préculture sans respiration (données non montrées). Lorsque la préculture était réalisée en conditions de respiration, la production chutait à 10,3µg/100g (données non montrées). Ainsi, pour la souche mère, la préculture en conditions de respiration avait un effet négatif sur la production de vitamine K2.

### B-V- L'influence de la dose d'ensemencement, de la population bactérienne finale et du pH du lait

Dans les conditions de « resting cells » décrites précédemment pour une préculture en respiration, la population bactérienne initiale s'élève à environ 10¹⁰UFC/mL.

Cette dose d'ensemencement étant difficilement applicable dans le cadre d'un procédé industriel, les Inventeurs ont étudié l'influence précise de la quantité initiale de cellules sur la production de vitamine K2 pendant la phase de fermentation en lait.

Par ailleurs, la quantité de cellules étant plus élevée dans une préculture en conditions de respiration que dans une préculture classique, les Inventeurs ont cherché à déterminer si le gain de production observé pour les précultures en conditions de respiration était dû à une simple augmentation de la dose d'ensemencement, ou bien à un apport spécifique du processus de respiration.

Les Inventeurs ont également cherché à déterminer si la population bactérienne finale, après la phase de « resting cells », jouait un rôle déterminant sur la quantité de vitamine K2 obtenue. Sachant qu'il existe une certaine mortalité bactérienne due, entre autres, à la diminution du pH pendant la fermentation, les Inventeurs ont étudié l'impact de l'utilisation d'un lait tamponné sur le niveau de production de vitamine K2.

Afin de répondre à ces différentes questions, des essais ont été réalisés en conditions de « resting cells », en lait classique ou tamponné, avec des doses d'ensemencement allant de 10⁶UFC/mL à 10¹⁰UFC/mL environ, et ce, à partir de précultures classiques ou en respiration.

Les précultures de la souche I-3558 ont été réalisées sur milieu M17 Lac à 30°C. Pour les expériences en conditions de respiration, le milieu a été additionné de 20µL/ml d'une solution d'hémine à 0,5mg/ml (dans de la soude 0,1M) et la préculture a été agitée pendant l'incubation sur une nuit. Différents volumes de ces précultures (voir Tableau IV ci-dessous) ont ensuite été centrifugés à 10000g pendant 10min.

**Tableau IV**

| Ensemencement | Volume préculture R+ | Volume préculture R- |
|---|---|---|
| 100% | 40mL | 160mL |
| 75% | 30mL | 120mL |
| 50% | 20mL | 80mL |
| 30% | 12mL | 48mL |
| 20% | 8mL | 32mL |
| 10% | 4mL | 16mL |
| 5% | 2mL | 8mL |
| 1% | 0,4mL | 1,6mL |
| 0,01% | 0,04mL | 0,16mL |

Le surnageant a été retiré et soit remplacé par 40mL de lait entier classique (précultures R+ et R-), soit additionné de β-glycérophosphate à 0,075M final (préculture R+ uniquement). Les échantillons ont ensuite été incubés à 30°C pendant 24h. Un aliquote a été prélevé pour effectuer un dénombrement puis les échantillons ont été congelés à -80°C pour un dosage ultérieur. Les résultats du dosage et des dénombrements réalisés avant et après la phase de « resting cells » sont indiqués dans le tableau V ci-dessous. Ce tableau fournit les résultats du dosage de vitamine K2 et des dénombrements effectués avant (T0) et après la phase de « resting cells » (Tf) à partir de pré-cultures classiques ou en respiration

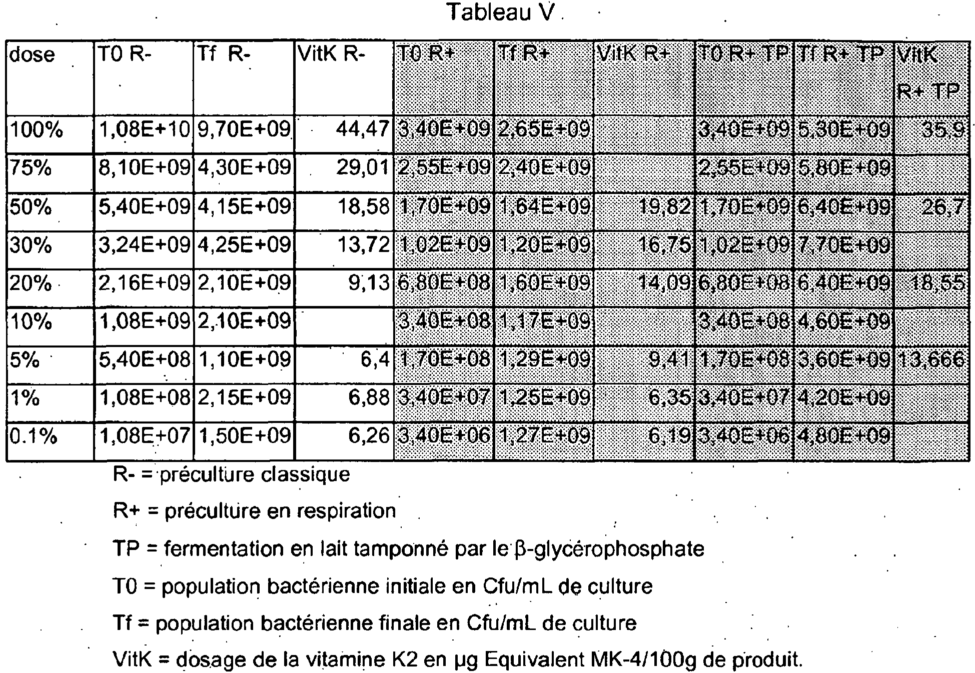

Les résultats obtenus sont représentés dans la figure 5. Y sont également représentés, à titre indicatif, les résultats obtenus avec une préculture en conditions de respiration, réalisée en fermenteur suivie d'une phase de « resting cells » en lait classique (courbe « respiration en fermenteur »). Les résultats sont comparables à ceux obtenus avec des précultures en tube (courbe « respiration labo »).

Les résultats obtenus (Tableau V et figure 5) indiquaient que :
- La teneur en vitamine K2 dépendait de la dose d'ensemencement ;
- La « pente » de la courbe était plus importante pour une préculture en conditions de respiration que pour une préculture classique. Ainsi, pour une population bactérienne donnée, la production de vitamine K2 était plus importante lorsque la préculture était réalisée en conditions de respiration. Le gain observé en conditions de respiration semblait donc dû à un apport spécifique du processus de respiration plutôt qu'à une simple augmentation de la dose d'ensemencement, ce qui a confirmé l'intérêt de cette piste ;
- La production de vitamine K2 était plus importante lorsque la phase de « resting cells » était réalisée en lait tamponné. Ceci pourrait être dû soit à une meilleure survie des bactéries dans ce milieu, puisque pour une même population initiale, la population finale était de 2 à 6 fois plus élevée en lait tamponné qu'en lait classique (voir tableau V), soit à une meilleure « extraction » de la vitamine K2 en lait tamponné.

### REFERENCES

Bolotin et al. 2001. Genome Research 11, 731-753
Duwat et al. 2001. J. Bacteriol. 183(15), 4509-16
Morishita et al. 1999. J. Dairy Sci. 82,1897-1903
Parker et al. 2003. Journal of Food Science 68(7), 2325-2330
Vido et al. 2005. J. Bact. 187, 601-10
Hart JP, et al. [letter]. Lancet. 1984;2:283
Hart JP, et al. J Clin Endocrinol Metab. 1985;60:1268-9
Hauschka PV, et al. Physiol Rev. 1989;69:990-1047
Ducy P, et al. Nature. 1996;382:448-52
Väänänen HK, et al. Calcif Tissue Int. 1999;64:S79
Ronden JE, et al. Biochim Biophys Acta. 1998;1379:16-22
Knapen MH, et al. Ann Intern Med. 1989 Dec 15;111(12):1001-5
Szulc P, et al. J Clin Invest. 1993 Apr;91 (4):1769-74
Booth SL, et al. Am J Clin Nutr. 2000;71:1201-8
Shiraki M, et al. J Bone Miner Res. 2000;15:515-21
Braam LAJLM, et al. Calcif Tissue Int. 2003 Jul;73(1):21-6
Hirano J and IshiiY. J Orthop Sci. 2002; 7:364-369.
Cocaign-Bousquet, M., et al. Journal of Applied Bacteriology 1995; 79, 108-116
Demain Al 1981. Science 214, 987-995

## Revendications

1. Procédé pour augmenter la quantité de vitamine K2 obtenue en cultivant au moins une souche de bactérie lactique productrice de vitamine K2, dans lequel la culture de ladite souche est réalisée dans des conditions de « resting cells », ledit procédé comprenant au moins :
a) la préculture de ladite souche dans des conditions de respiration, dans un milieu de pré-culture approprié contenant au moins une porphyrine à une concentration finale d'au moins 0,5 µg/ml;
b) l'ensemencement d'un milieu de culture approprié contenant au moins 0.5% de matière grasse avec une quantité de cellules bactériennes vivantes allant de 10⁸ à 10¹¹ ufc_{/}ml environ ; et
c) la fermentation du milieu ainsi ensemencé pendant une période allant de 4 à 48 heures, à une température de 4 à 50°C, de sorte qu'à l'issue de l'étape c), la quantité de vitamine K2 produite par la culture en « resting cells » est supérieure, d'un facteur au moins égal à environ 1,2 à celle obtenue en cultivant ladite souche dans des conditions de fermentation standard.

2. Procédé selon la revendication 1, **caractérisé en ce que** ledit milieu de culture approprié contient au moins 1.5% de matière grasse.

3. Procédé selon la revendication 1 ou 2, **caractérisé en ce que** ledit milieu de culture approprié est un lait ou un lait dont le pouvoir tampon a été augmenté.

4. Procédé selon l'une quelconque des revendications 1 à 3, **caractérisé en ce que** ladite souche de bactérie lactique productrice de vitamine K2 est choisie parmi les genres *Lactococcus, Leuconostoc, Enterococcus* et *Propionibacterium.*

5. Procédé selon la revendication 4, **caractérisé en ce que** ladite souche de bactérie lactique est choisie parmi les espèces *Lactococcus lactis, Leuconostoc lactis, Leuconostoc pseudomesenteroides, Leuconostoc mesenteroides, Leuconostoc dextranicum, Enterococcus faecium, Propionibacterium sp.*

6. Procédé selon la revendication 5, **caractérisé en ce que** ladite souche de bactérie lactique est choisie parmi les variants naturels de *Lactococcus lactis subsp. Cremoris* producteurs de vitamine K2 :
- 1-3557 déposé à la Collection Nationale de Culture des Microorganismes (CNCM, Institut Pasteur, 25 rue du Docteur Roux, 75724 Paris cedex 15, France) le 20/01/2006,
- 1-3558 déposé à la CNCM, Institut Pasteur, 25 rue du Docteur Roux, Paris cedex 15, France le 20/01/2006, et
- 1-3626 déposé à la CNCM, Institut Pasteur, 25 rue du Docteur Roux, 75724 Paris cedex 15, France le 19/06/2006.

7. Procédé pour produire de la vitamine K2, comprenant au moins :
a) la mise en oeuvre du procédé selon l'une quelconque des revendications 1 à 6 ; et
b) la récupération de la vitamine K2 ainsi produite.

8. Procédé pour préparer un produit alimentaire enrichi en vitamine K2, comprenant au moins:
a) la production de vitamine K2 selon le procédé de la revendication 7 ;
b) l'ajout de la vitamine K2 ainsi produite audit produit alimentaire, ou à une préparation intermédiaire de celui-ci ; et
c) l'obtention dudit produit alimentaire enrichi en vitamine K2.

9. Procédé pour préparer un produit alimentaire enrichi en vitamine K2, comprenant au moins :
a) la culture d'au moins une souche de bactérie lactique productrice de vitamine K2 dans des conditions de « resting cells » conformément au procédé selon l'une quelconque des revendications 1 à 6 ;
b) l'ajout de la biomasse obtenue à partir de la culture sous a), audit produit alimentaire ou à une préparation intermédiaire de celui-ci ; et
c) l'obtention dudit produit alimentaire enrichi en vitamine K2.

## Patentansprüche

1. Verfahren zum Erhöhen der Menge von Vitamin K2, die erhalten wird, indem man mindestens einen Stamm von Milchsäurebakterien, welcher Vitamin K2 produziert, kultiviert, in welchem die Kultivierung des Stamms unter "resting cells"-Bedingungen ausgeführt wird, wobei das Verfahren mindestens umfasst:
a) die Vorkultivierung des Stamms unter Atmungsbedingungen in einem geeigneten Vorkultivierungsmedium, welches mindestens ein Porphyrin in einer Endkonzentration von mindestens 0,5 µg/ml enthält;
b) das Beimpfen eines geeigneten Kulturmediums, welches mindestens 0,5% Fett enthält, mit einer Menge von lebenden Bakterienzellen, die von etwa 10⁸ bis 10¹¹ KBE/ml reicht; und
c) die Fermentation des so beimpften Mediums während einer Zeitspanne, die von 4 bis 48 Stunden reicht, bei einer Temperatur von 4 bis 50°C derart, dass am Ende des Schritts c) die durch die Kultivierung mittels "resting cells" produzierte Menge von Vitamin K2 um einen Faktor von mindestens etwa 1,2 höher ist als jene, die erhalten wird, indem man den Stamm unter Standard-Fermentationsbedingungen kultiviert.

2. Verfahren nach Anspruch 1, **dadurch gekennzeichnet, dass** das geeignete Kulturmedium mindestens 1,5% Fett enthält.

3. Verfahren nach Anspruch 1 oder 2, **dadurch gekennzeichnet, dass** das geeignete Kulturmedium eine Milch oder eine Milch, deren Pufferungsvermögen erhöht worden ist, ist.

4. Verfahren nach einem der Ansprüche 1 bis 3, **dadurch gekennzeichnet, dass** der Stamm von Milchsäurebakterien, welcher Vitamin K2 produziert, unter den Gattungen *Lactococcus, Leuconostoc, Enterococcus* und *Propionibacterium* ausgewählt wird.

5. Verfahren nach Anspruch 4, **dadurch gekennzeichnet, dass** der Stamm von Milchsäurebakterien unter den Arten *Lactococcus lactis, Leuconostoc lactis, Leuconostoc pseudomesenteroides, Leuconostoc mesenteroides, Leuconostoc dextranicum, Enterococcus faecium, Propionibacterium sp.* ausgewählt wird.

6. Verfahren nach Anspruch 5, **dadurch gekennzeichnet, dass** der Stamm von Milchsäurebakterien unter den Vitamin K2 produzierenden, natürlichen Varianten von *Lactococcus lactis subsp. cremoris*:
- I-3557, die bei der Collection Nationale de Culture des Microorganismes (CNCM, Institut Pasteur, 25 rue du Docteur Roux, 75724 Paris cedex 15, Frankreich) am 20.01.2006 hinterlegt worden ist,
- I-3558, die bei der CNCM, Institut Pasteur, 25 rue du Docteur Roux, Paris cedex 15, Frankreich, am 20.01.2006 hinterlegt worden ist,
- I-3626, die bei der CNCM, Institut Pasteur, 25 rue du Docteur Roux, 75724 Paris cedex 15, Frankreich, am 19.06.2006 hinterlegt worden ist,
ausgewählt wird.

7. Verfahren zum Herstellen von Vitamin K2, welches mindestens umfasst:
a) das Ausführen des Verfahrens nach einem der Ansprüche 1 bis 6 und
b) die Gewinnung des so hergestellten Vitamins K2.

8. Verfahren zum Herstellen eines mit Vitamin K2 angereicherten Nahrungsmittelerzeugnisses, welches mindestens umfasst:
a) die Herstellung von Vitamin K2 gemäß dem Verfahren des Anspruchs 7;
b) das Hinzufügen des so hergestellten Vitamins K2 zu dem Nahrungsmittelerzeugnis oder zu einer intermediären Zubereitung von jenem und
c) die Gewinnung des mit Vitamin K2 angereicherten Nahrungsmittelerzeugnisses.

9. Verfahren zum Herstellen eines mit Vitamin K2 angereicherten Nahrungsmittelerzeugnisses, welches mindestens umfasst:
a) das Kultivieren von mindestens einem Stamm von Milchsäurebakterien, welcher Vitamin K2 produziert, unter "resting cells"-Bedingungen entsprechend dem Verfahren nach einem der Ansprüche 1 bis 6;
b) das Hinzufügen der ausgehend von der Kultivierung unter a) erhaltenen Biomasse zu dem Nahrungsmittelerzeugnis oder einer intermediären Zubereitung von jenem; und
c) die Gewinnung des mit Vitamin K2 angereicherten Nahrungsmittelerzeugnisses.

## Claims

1. A method to increase the quantity of vitamin K2 obtained by cultivating at least one strain of lactic acid bacterium that produces vitamin K2, wherein said strain is cultured in resting-cell conditions, said method comprising at least:
a) pre-culture of said strain under respiration conditions, in a suitable pre-culture medium containing at least one porphyrin at a final concentration of at least 0.5 µg/ml;
b) inoculation of a suitable culture medium containing at least 0.5% fat with a quantity of living bacterial cells ranging from about 10⁸ to 10¹¹ CFU/ml; and
c) fermentation of the medium thus inoculated for a period ranging from 4 hours to 48 hours, at a temperature of 4°C to 50°C, in such a way that, at the end of step c), the quantity of vitamin K2 produced by the resting-cell culture is higher, by a factor at least equal to about 1.2, than that obtained by cultivating said strain under standard fermentation conditions.

2. The method according to claim 1, **characterised in that** said suitable culture medium contains at least 1.5% fat.

3. The method according to claim 1 or 2, **characterised in that** said suitable culture medium is a milk or milk whose buffering capacity has been increased.

4. The method according to any one of claims 1 to 3, **characterised in that** said strain of lactic acid bacterium that produces vitamin K2 is selected from the genera *Lactococcus, Leuconostoc, Enterococcus* and *Propionibacterium.*

5. The method according to claim 4, **characterised in that** said strain of lactic acid bacterium is selected from the species *Lactococcus lactis, Leuconostoc lactis, Leuconostoc pseudomesenteroides, Leuconostoc mesenteroides, Leuconostoc dextranicum, Enterococcus faecium,* and *Propionibacterium* sp.

6. The method according to claim 5, **characterised in that** said strain of lactic acid bacterium is selected from the natural variants of *Lactococcus lactis* subsp. *cremoris* that produce vitamin K2:
- 1-3557 filed with France's *Collection Nationale de Culture des Microorganismes* (CNCM, Institut Pasteur, 25 rue du Docteur Roux, 75724 Paris cedex 15, France) on 20/01/2006,
- 1-3558 filed with the CNCM, Institut Pasteur, 25 rue du Docteur Roux, 75724 Paris cedex 15, France on 20/01/2006, and
- 1-3626 filed with the CNCM, Institut Pasteur, 25, rue du Docteur Roux, 75724 Paris cedex 15, France on 19/06/2006.

7. A method for producing vitamin K2, comprising at least:
a) implementation of the method according to any one of claims 1 to 6; and
b) recovery of the vitamin K2 thus produced.

8. A method for preparing a food product enriched in vitamin K2, comprising at least:
a) producing vitamin K2 according to the method of claim 7;
b) adding the vitamin K2 thus produced to said food product, or to an intermediate preparation of the same; and
c) obtaining said food product enriched in vitamin K2.

9. A method for preparing a food product enriched in vitamin K2, comprising at least:
a) culturing at least one strain of lactic acid bacterium that produces vitamin K2 under resting-cell conditions in accordance with the method according to any one of claims 1 to 6;
b) adding the biomass obtained from the culture in step a) to said food product or to an intermediate preparation of the same; and
c) obtaining said food product enriched in vitamin K2.
